# EUROPEAN PATENT APPLICATION

(11) **EP 4 316 395 A1**
(43) Date of publication of application: **07.02.2024**
(21) Application number: 21935335.6
(22) Date of filing: 31.12.2021
(51) Int. Cl.: A61B 17/34, A61B 17/3205, A61F 5/00, A61B 90/00, A61B 17/32, A61B 17/00

(54) **SLEEVE GASTRECTOMY AID DEVICE**

(30) Priority: 31.03.2021 KR 20210042250
(71) Applicant: Ajou University Industry-Academic Cooperation Foundation, Gyeonggi-do 16499 (KR); Soonchunhyang University Industry Academy Cooperation Foundation, Asan-si, Chungcheongnam-do 31538 (KR)
(72) Inventor: LEE, Moon Gu, Suwon-si, Gyeonggi-do 16499 (KR); KIM, Sang Hyun, Seoul 05287 (KR); YUN, Sang Chul, Seoul 04069 (KR); CHO, Sung Woo, Seoul 05502 (KR); JUNG, Chang Ho, Seoul 05698 (KR); KANG, Young Jae, Gwangju 62250 (KR)
(74) Representative: Zacco Sweden AB
(86) International application number: PCT/KR2021/020348
(87) International publication number: WO 2022/211222

(57) **Abstract**

The present disclosure relates to a sleeve gastrectomy aid device including: a guide configured to be inserted into a stomach of a patient; an expander configured to be disposed outside the guide and have a volume that changes according to an amount of air therein; a first measurer configured to measure a pressure applied to the expander and have a shape that changes in accordance with a change in the volume of the expander; and a monitor configured to be connected to the first measurer and provide resection position information through a value of the pressure measured by the first measurer.

## Description

### [Technical Field]

This application claims priority to and the benefit of Korean Patent Application No. 10-2021-0042250, filed on March 31, 2020, the disclosure of which is incorporated herein by reference in its entirety.

The present disclosure relates to a sleeve gastrectomy aid device, and more particularly, to a sleeve gastrectomy aid device inserted into the stomach to aid resection surgery.

### [Background Art]

Generally, sleeve gastrectomy is a typical surgical method for severe obesity. Sleeve gastrectomy is a laparoscopic surgery in which the stomach is cut using special tools such as a camera for observation, a stapler, and a traction device, leaving only a thin, constant-diameter stomach with a volume of 100 to 150 ml. Unlike other surgeries, sleeve gastrectomy is surgery to simply reduce the size of the stomach and directly accesses the internal organs without an external incision, thus allowing a quick post-surgery recovery. As compared to other surgical methods, sleeve gastrectomy has advantages that the user's burden is low and the complication rate is the lowest. Sleeve gastrectomy is performed by inserting a bougie into the lumen of the stomach through the mouth and cutting the stomach using a stapler and a traction device according to the shape of the bougie.

However, conventional surgical tools for sleeve gastrectomy do not have a means to quantitatively provide necessary information during resection, and thus the process of cutting and pulling the stomach depends on the user's skill without a set standard, and there is a problem that there is a large difference between the shape of the stomach remaining after surgery and the target shape of the stomach. When the volume of the remaining stomach is too small, gastroesophageal reflux occurs as a side effect, and when the volume of the remaining stomach is too large, there is a problem that a patient may fail to reduce weight or the patient's weight may increase again.

The related art of the present disclosure is disclosed in Korean Unexamined Patent Application Publication No. 10-2020-0103489 (Date of Publication: September 2, 2020, Title of Disclosure: Inserting tube for gastric resection guides).

### [Disclosure]

### [Technical Problem]

The present disclosure is directed to providing a sleeve gastrectomy aid device capable of controlling a volume corresponding to the shape of the remaining stomach.

The present disclosure is also directed to providing a sleeve gastrectomy aid device capable of quantitatively providing information on a resection state.

### [Technical Solution]

The present disclosure provides a sleeve gastrectomy aid device including: a guide configured to be inserted into a stomach of a patient; an expander configured to be disposed outside the guide and have a volume that changes according to an amount of air therein; a first measurer configured to measure a pressure applied to the expander and have a shape that changes in accordance with a change in volume of the expander; and a monitor configured to be connected to the first measurer and provide resection position information through a value of the pressure measured by the first measurer.

Also, the first measurer may include: a plurality of first measuring members configured to be attached to an inner side surface of the expander; and a connector configured to be connected to the first measuring members adjacent to each other and expand and contract according to a distance between the first measuring members adjacent to each other.

Also, the first measurer may extend in a longitudinal direction of the expander and may be provided as a plurality of first measurers, and the plurality of first measurers may be spaced apart in a circumferential direction of the expander.

Also, the plurality of first measuring members provided in each of the first measurers may be disposed to be spaced apart at predetermined intervals in the longitudinal direction of the expander.

Also, the first measuring members provided in the first measurers, which are different from each other, may be disposed along the same circumference of the expander.

Also, the connector may extend in a zigzag shape in a longitudinal direction thereof.

Also, the connector may include a plurality of connecting members connected to each other in a lattice form and each having a diameter expanding in a direction parallel to a direction in which an external force is applied.

Also, the connector may be made of a material capable of conducting current.

Also, the resection position information may be information on a distance from the expander to a resection tool.

Also, the monitor may include: a controller configured to determine the resection position information based on the value of the pressure measured by the first measurer; and a display configured to display the information determined by the controller to the outside.

Also, the controller may determine resection position sections according to a magnitude of the value of the pressure measured by the first measurer.

Also, the resection position sections may include: an approach-necessary section indicating a case in which the magnitude of the pressure measured by the first measurer is lower than or equal to a first set pressure; a separation-necessary section indicating a case in which the magnitude of the pressure measured by the first measurer is higher than or equal to a second set pressure; and a resection-possible section indicating that the magnitude of the pressure measured by the first measurer is between the first set pressure and the second set pressure.

Also, the display may display different colors according to the resection position sections.

The present disclosure also provides a sleeve gastrectomy aid device including: a guide configured to be inserted into a stomach of a patient; an expander configured to be disposed outside the guide and have a volume that changes according to an amount of air therein; a second measurer configured to measure an air pressure inside the expander; and a monitor configured to be connected to the second measurer and provide resection position information through a value of the pressure measured by the second measurer.

Also, the second measurer may be connected to an injector configured to inject air into the expander.

Also, the resection position information may be information on a distance from the expander to a resection tool.

Also, the monitor may include: a controller configured to determine the resection position information based on the value of the pressure measured by the second measurer; and a display configured to display the information determined by the controller to the outside.

Also, the controller may determine resection position sections according to a magnitude of the value of the pressure measured by the second measurer.

Also, the resection position sections may include: an approach-necessary section indicating a case in which the magnitude of the pressure measured by the second measurer is lower than or equal to a first set pressure; a separation-necessary section indicating a case in which the magnitude of the pressure measured by the second measurer is higher than or equal to a second set pressure; and a resection-possible section indicating that the magnitude of the pressure measured by the second measurer is between the first set pressure and the second set pressure.

Also, the display may display different colors according to the resection position sections.

### [Advantageous Effects]

In a sleeve gastrectomy aid device according to the present disclosure, since a guide is provided so that its shape can be freely changed, the insertion of the guide into the stomach can be smoothly performed.

Also, in the sleeve gastrectomy aid device according to the present disclosure, since the volume of an expander can be changed according to the shape and size of the stomach, suitable surgery can be performed for each patient.

Also, in the sleeve gastrectomy aid device according to the present disclosure, since the shape of a first measurer can be changed, damage such as breakage due to a change in volume of the expander can be prevented.

Also, in the sleeve gastrectomy aid device according to the present disclosure, since a plurality of first measuring members are provided and can separately measure the pressure applied to each position of the expander, a more accurate pressure value can be measured.

Also, in the sleeve gastrectomy aid device according to the present disclosure, since a second measurer is provided to measure the pressure applied to the expander from outside the expander, the second measurer can be easily installed and managed.

Also, in the sleeve gastrectomy aid device according to the present disclosure, since a controller provides quantitative information through pressure inside the stomach that is measured by the first measurer or the second measurer, an operator can perform surgery in a consistent manner regardless of the operator's skill.

In addition, in the sleeve gastrectomy aid device according to the present disclosure, since a display displays different colors according to a resection position section, resection position information and resection length information can be provided to be more intuitively identifiable.

### [Description of Drawings]

FIG. 1 is an installation state diagram schematically illustrating an installation state of a sleeve gastrectomy aid device according to one embodiment of the present disclosure.
FIG. 2 is a perspective view schematically illustrating a configuration of the sleeve gastrectomy aid device according to one embodiment of the present disclosure.
FIG. 3 is a lateral view schematically illustrating the configuration of the sleeve gastrectomy aid device according to one embodiment of the present disclosure.
FIGS. 4A and 4B are enlarged views schematically illustrating a configuration of a connector according to one embodiment of the present disclosure.
FIGS. 5A and 5B are enlarged views schematically illustrating a configuration of a connector according to another embodiment of the present disclosure.
FIG. 6 is a flowchart schematically illustrating a configuration of a monitor according to one embodiment of the present disclosure.
FIG. 7 is a view schematically illustrating resection position sections determined by a controller according to one embodiment of the present disclosure.
FIGS. 8 and 9 are operation diagrams schematically illustrating an operation process of the sleeve gastrectomy aid device according to one embodiment of the present disclosure.
FIGS. 10A and 10B are operation diagrams schematically illustrating operation states of the controller in the process of inserting a guide according to one embodiment of the present disclosure.
FIGS. 11A and 11B are operation diagrams schematically illustrating operation states of the controller in an approach-necessary section according to one embodiment of the present disclosure.
FIGS. 12A and 12B are operation diagrams schematically illustrating operation states of the controller in a separation-necessary section according to one embodiment of the present disclosure.
FIGS. 13A and 13B are operation diagrams schematically illustrating operation states of the controller in a resection-possible section according to one embodiment of the present disclosure.
FIG. 14 is a view illustrating a state in which an expander is in close contact with an inner wall of the stomach according to one embodiment of the present disclosure.
FIG. 15 is a view illustrating pressure values measured by a plurality of first measuring members disposed along the same circumference of the expander in the state of FIG. 14.
FIG. 16 is a view illustrating a state in which the expander is not in close contact with the inner wall of the stomach according to one embodiment of the present disclosure.
FIG. 17 is a view illustrating pressure values measured by the plurality of first measuring members disposed along the same circumference of the expander in the state of FIG. 16.
FIG. 18 is a perspective view schematically illustrating a configuration of a sleeve gastrectomy aid device according to another embodiment of the present disclosure.
FIG. 19 is a flowchart schematically illustrating a configuration of a monitor according to another embodiment of the present disclosure.
FIGS. 20A and 20B are operation diagrams schematically illustrating operation states of a controller in the process of inserting a guide according to another embodiment of the present disclosure.
FIGS. 21A and 21B are operation diagrams schematically illustrating operation states of the controller in an approach-necessary section according to another embodiment of the present disclosure.
FIGS. 22A and 22B are operation diagrams schematically illustrating operation states of the controller in a separation-necessary section according to another embodiment of the present disclosure.
FIGS. 23A and 23B are operation diagrams schematically illustrating operation states of the controller in a resection-possible section according to another embodiment of the present disclosure.

### [Modes of the Invention]

Hereinafter, embodiments of a sleeve gastrectomy aid device according to the present disclosure will be described with reference to the accompanying drawings.

In this process, thicknesses of lines or sizes of elements illustrated in the drawings may be exaggerated for clarity and convenience of description. Also, terms used below are terms defined in consideration of functions in the present disclosure and may vary according to an intention or customary practice of a user or an operator. Therefore, the terms should be defined based on the content throughout the specification.

Also, in this specification, when a certain portion is described as being "connected (or linked)" to another portion, this may include not only a case in which the portion is "directly connected (or linked)" to the other portion but also a case in which the portion is "indirectly connected (or linked)" to the other portion while another member is disposed therebetween. In this specification, when a certain portion is described as "including (or having)" a certain element, unless particularly described otherwise, this means that the certain portion may further "include (or have)" other elements instead of excluding other elements.

Also, throughout the specification, like reference numerals may refer to like elements. Even when not mentioned or described with reference to specific drawings, like or similar reference numerals may be described based on other drawings. Also, parts not denoted by reference numerals in specific drawings may be described based on other drawings. Also, the number, shape, and size of specific elements, a relative difference between the sizes of the elements, and the like included in the drawings of the present application are set for convenience of understanding and may be implemented in various other forms while not limiting the embodiments.

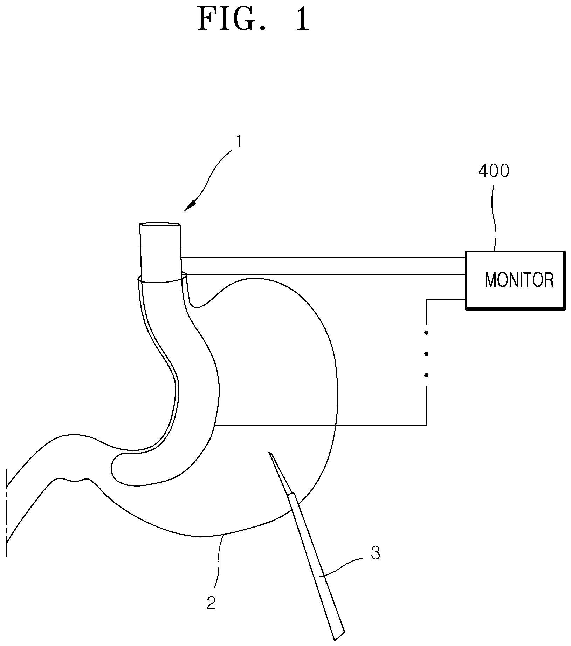

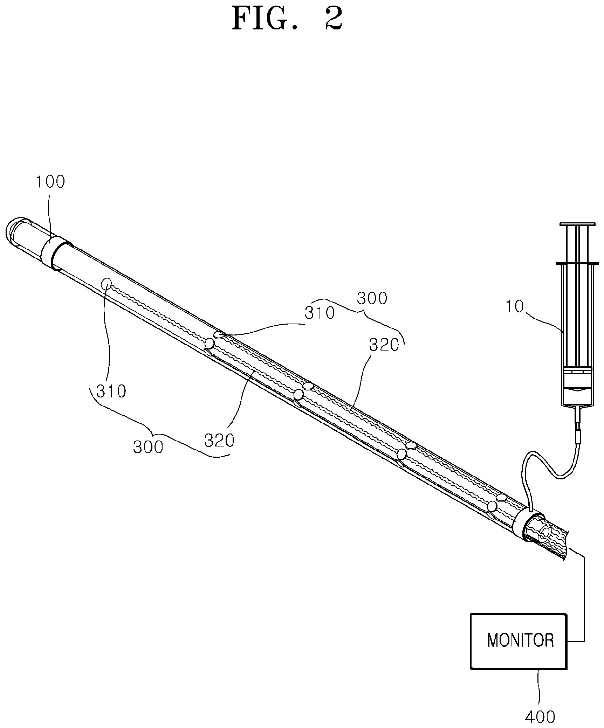

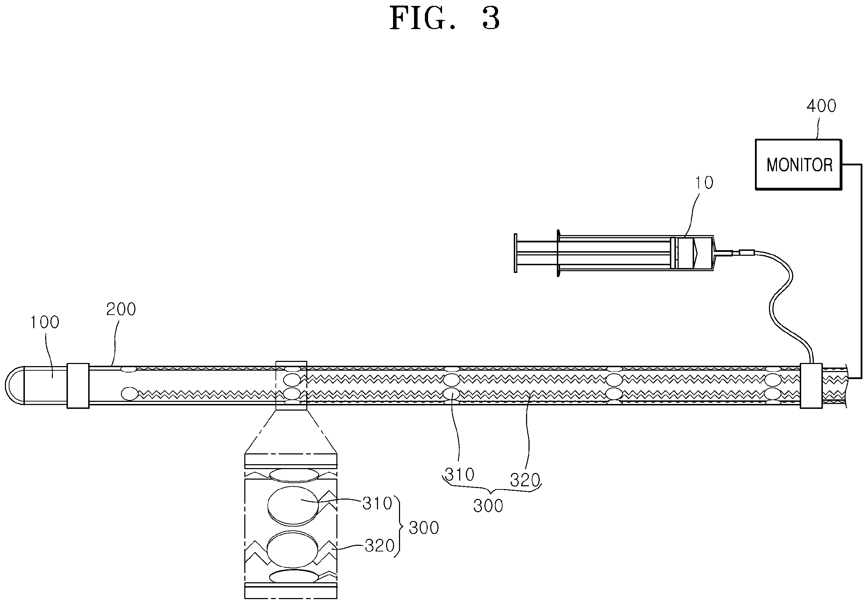

FIG. 1 is an installation state diagram schematically illustrating an installation state of a sleeve gastrectomy aid device according to one embodiment of the present disclosure, FIG. 2 is a perspective view schematically illustrating a configuration of the sleeve gastrectomy aid device according to one embodiment of the present disclosure, and FIG. 3 is a lateral view schematically illustrating the configuration of the sleeve gastrectomy aid device according to one embodiment of the present disclosure.

Referring to FIGS. 1 to 3, a sleeve gastrectomy aid device 1 according to one embodiment of the present disclosure includes a guide 100, an expander 200, a first measurer 300, and a monitor 400.

During sleeve gastrectomy, the guide 100 is inserted into a stomach 2 via the esophagus through an oral cavity or nasal cavity of a patient. The guide 100 supports the expander 200, which will be described below, and guides insertion of the expander 200 into the stomach 2. The guide 100 may include a flexible material such as silicone or synthetic resin so that the shape of the guide 100 may be changed. Accordingly, the insertion of the guide 100 into the stomach 2 can be more smoothly performed, and by being bent corresponding to the curvature, shape, or the like of the stomach, the guide 100 can roughly determine the form of the stomach 2 remaining after the resection surgery.

The guide 100 according to one embodiment of the present disclosure is formed to have a tubular shape including a silicone material. A diameter of the guide 100 may be formed to have a smaller value than a diameter of the patient's esophagus. The length of the guide 100 may be changed to various other values within the range of length that allows the guide 100 to be inserted into the stomach 2. An end of the guide 100 is formed to be convex with a predetermined curvature, and thus the insertion of the guide 100 into the stomach 2 can be more smoothly performed.

The expander 200 is installed outside the guide 100, and the volume of the expander 200 changes as the expander 200 expands or contracts according to the amount of air therein. As the volume of the expander 200 is controlled so that the expander 200 has a predetermined volume inside the stomach 2, the expander 200 determines the volume of the stomach 2 remaining after resection. The expander 200 may include a natural resin material such as rubber or latex having flexibility or a synthetic resin material such as chloroprene having flexibility. The expander 200 according to one embodiment of the present disclosure is formed in the form of a cylindrical rubber membrane and disposed to surround an outer peripheral surface of the guide 100. Both ends of the expander 200 are fixed to the outer peripheral surface of the guide 100 by a ring-shaped fixer. The expander 200 communicates with an injector 10 and receives air injected thereinto. As air is injected into the expander 200, the volume of the expander 200 expands outwardly from the guide 100. In this case, like a balloon, the expander 200 expands in a form in which a distance between two points arbitrarily set on a surface between both ends of the expander 200, which are in close contact with the outer peripheral surface of the guide 100, increases.

The injector 10 is provided to communicate with the expander 200 and supply air into the expander 200 or discharge air from inside the expander 200. The injector 10 according to one embodiment of the present disclosure may include a hose member formed to have the shape of a hollow tube and have one side communicating with the inside of the expander 200 and an injecting member formed in the form of a syringe, an air pump, or the like and connected to the other side of the hose member to inject air into the expander 200 or discharge air from inside the expander 200.

The first measurer 300 is disposed inside the expander 200 to measure a value of pressure applied to the expander 200. More specifically, the first measurer 300 measures pressure applied to the expander 200 from outside the expander 200 as a resection tool 3 presses the stomach 2 during resection surgery. The first measurer 300 is connected to the monitor 400, which will be described below, and transmits a measured pressure value to the monitor 400. The first measurer 300 is provided so that its shape may be changed in accordance with a change in volume of the expander 200. Accordingly, through changing of its shape, the first measurer 300 may absorb an external force, such as tension, applied when the volume of the expander 200 changes. The first measurer 300 may be formed in a form that extends in the longitudinal direction of the expander 200. The first measurer 300 may be provided as a plurality of first measurers 300, and the plurality of first measurers 300 may be disposed to be spaced apart at predetermined intervals in the circumferential direction of the expander 200. In this case, the first measurers 300 may extend different lengths.

The first measurer 300 according to one embodiment of the present disclosure includes a first measuring member 310 and a connector 320.

The first measuring member 310 is attached to an inner side surface of the expander 200 to measure the pressure applied to the expander 200. The first measuring member 310 may be provided as a plurality of first measuring members 310, and the plurality of first measuring members 310 may separately measure the pressure applied to the expander 200. Accordingly, the first measuring members 310 may more accurately measure a value of pressure applied to each position of the expander 200. The first measuring member 310 according to one embodiment of the present disclosure may be formed in the form of a contact type pressure sensor such as a load cell configured to output an electrical signal according to a value of pressure applied from outside the expander 200. The first measuring member 310 may be formed in the form of a thin film having flexibility so that the first measuring member 310 may come in close contact with the inner side surface of the expander 200 regardless of the curvature or the like of the expander 200.

The plurality of first measuring members 310 provided in each of the first measurers 300 may be disposed to be spaced apart at predetermined intervals in the longitudinal direction of the expander 200. In this case, the plurality of first measuring members 310 provided in each of the first measurers 300 may be spaced apart at equal intervals, and the first measuring members 310 provided in different first measurers 300 may be disposed along the same circumference of the expander 200. That is, as illustrated in FIG. 3, the plurality of first measuring members 300 may be disposed to form ring shapes on different circumferences of the expander 200 that are spaced apart in the longitudinal direction of the expander 200. Accordingly, the first measuring members 310 may allow the monitor 400, which will be described below, to determine whether the expander 200 is in close contact with the inner wall of the stomach 2.

The connector 320 is connected to adjacent first measuring members 310. The connector 320 is made of a material capable of conducting current, such as copper, and electrically connects adjacent first measuring members 310 and transmits an electrical signal output from the first measuring member 310 to the monitor 400, which will be described below. The connector 320 is provided as a plurality of connectors 320, and the plurality of connectors 320 sequentially connect the first measuring members 310 disposed to be spaced apart at predetermined intervals in the longitudinal direction of the expander 200. More specifically, the connectors 320 excluding the rearmost connector 320 have both ends connected to a pair of first measuring members 310, and the rearmost connector 320 has one end connected to the rearmost first measuring member 310 and the other end connected to the monitor 400. The plurality of connectors 320 may be disposed in a direction parallel to the longitudinal direction of the expander 200.

The connector 320 is provided to expand and contract according to a distance between adjacent first measuring members 310. More specifically, the connector 320 is provided to, by expanding and contracting, absorb a change in distance between adjacent first measuring members 310 caused by a change in volume of the expander 200. Accordingly, the connector 320 may be prevented from breaking due to tension applied as the distance between adjacent first measuring members 310 increases.

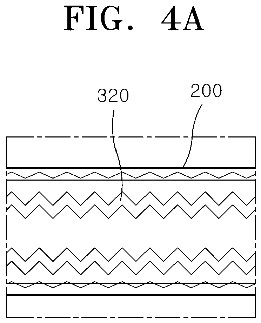

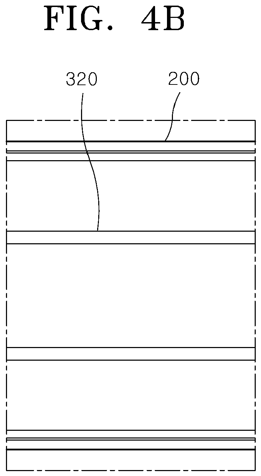

FIGS. 4A and 4B are enlarged views schematically illustrating a configuration of a connector according to one embodiment of the present disclosure.

Referring to FIGS. 4A and 4B, the connector 320 may be formed in the form of a wire extending in a zigzag shape in the longitudinal direction. More specifically, before the volume of the expander 200 expands, the connector 320 is compressed in a zigzag shape and pre-accumulates a length to which the connector 320 can extend, and as the volume of the expander 200 expands, the connector 320 extends in a straight line, and the length of the connector 320 is extended. Accordingly, the connector 320 may smoothly perform expanding and contracting without occupying an excessive volume.

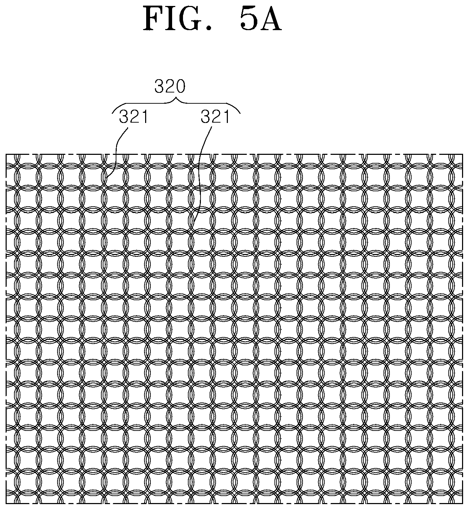

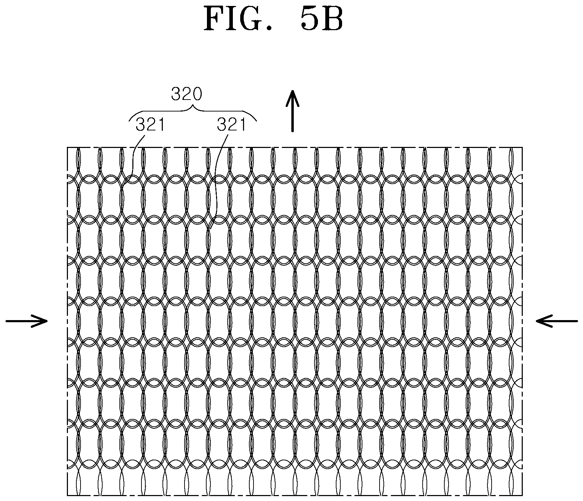

FIGS. 5A and 5B are enlarged views schematically illustrating a configuration of a connector according to another embodiment of the present disclosure.

Referring to FIGS. 5A and 5B, a connector 320 according to another embodiment of the present disclosure may include a connecting member 321.

The connecting member 321 is formed so that a diameter thereof may expand in a direction parallel to a direction in which an external force is applied. The connecting member 321 is provided as a plurality of connecting members 321, and the plurality of connecting members 321 are connected to each other in a lattice form. The connecting member 321 according to one embodiment of the present disclosure is formed in the shape of a ring-shaped wire. As the distance between adjacent first measuring members 310 increases, in a case in which an external force is applied in one direction (the up-down direction based on FIG. 5), the diameter of the connecting member 321 expands in the corresponding direction, and the diameter is reduced in a direction perpendicular to the corresponding direction. The plurality of connecting members 321 are connected in a shape formed by repetition of a form in which four different connecting members 321 are entangled at the top, bottom, left, and right based on any one connecting member 321. The plurality of connecting members 321 are electrically connected to each other in order to be able to transmit an electrical signal output from the first measuring member 310.

The monitor 400 is connected to the first measurer 300, determines resection position information through a pressure value measured by the first measurer 300, and provides the determined resection position information to the user. Here, an example of the resection position information may be information on a distance from the expander 200 to the resection tool 3.

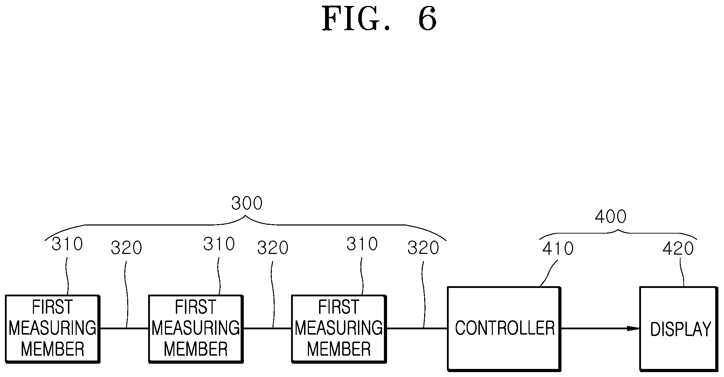

FIG. 6 is a flowchart schematically illustrating a configuration of a monitor according to one embodiment of the present disclosure.

Referring to FIG. 6, the monitor 400 according to one embodiment of the present disclosure includes a controller 410 and a display 420.

The controller 410 determines resection position information based on a pressure value measured by the first measurer 300. More specifically, the controller 410 converts the pressure value measured by the first measurer 300 into information on the distance from the expander 200 to the resection tool 3 to determine the resection position information of the resection tool 3. That is, by determining resection position sections D according to the magnitude of the pressure value measured by the first measurer 300, the controller 410 determines the resection position information. An example of the controller 410 according to one embodiment of the present disclosure may be a microprocessor, a computer, or the like connected to the first measuring member 310 to receive the measured pressure value from the first measuring member 310. The controller 410 may receive the measured pressure value from the first measuring member 310 via a wire through the connector 320. In this case, the controller 410 may use an average of pressure values measured by the plurality of first measuring members 310 to determine the resection position information of the resection tool 3.

The resection position sections D refer to sections obtained by dividing pressure values according to the magnitudes thereof, based on a value of an initial pressure P0 inside the stomach 2 that is measured by the first measurer 300 right after insertion of the guide 100. More specifically, the resection position sections D refer to sections between a pair of different pressure values among sections of pressures measured by the first measurer 300 as the position at which the stomach 2 is pressed or resected by the resection tool 3 is changed after the insertion of the guide 100.

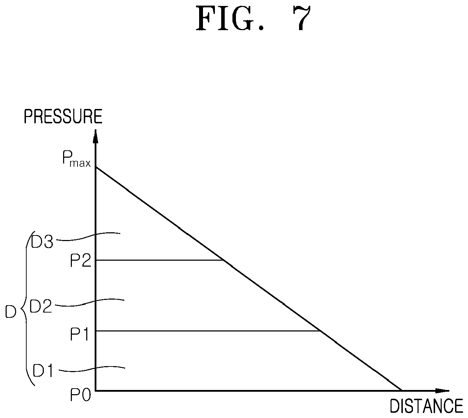

FIG. 7 is a view schematically illustrating the resection position sections determined by the controller according to one embodiment of the present disclosure.

Referring to FIG. 7, the resection position sections D according to one embodiment of the present disclosure include an approach-necessary section D1, a resection-possible section D2, and a separation-necessary section D3.

The approach-necessary section D1 indicates a case in which the magnitude of the pressure measured by the first measurer 300 is the initial pressure P0 or higher and a first set pressure P1 or lower. Here, the first set pressure P1 is a pressure value measured at the maximum distance among distances from the expander 200 to the resection tool 3 that allow the user to form the remaining stomach 2 with a target size. The first set pressure P1 may be changed in various ways according to the shape, size, and the like of the stomach 2. That is, the approach-necessary section D1 corresponds to a section in which the controller 410 determines, through the pressure value measured by the first measurer 300, that the resection tool 3 is excessively far away from the expander 200.

The separation-necessary section D3 indicates a case in which the magnitude of the pressure measured by the first measurer 300 is a second set pressure P2 or higher. Here, the second set pressure P2 is a pressure value measured at the minimum distance among the distances from the expander 200 to the resection tool 3 that allow the user to form the remaining stomach 2 with a target size. The second set pressure P2 may be changed in various ways according to the shape, size, and the like of the stomach 2. More specifically, the second set pressure P2 indicates a pressure lower than or equal to the maximum pressure that can be measured by the first measurer 300 or a pressure Pmax at which the mucosa of the stomach 2 ruptures. That is, the separation-necessary section D3 corresponds to a section in which the controller 410 determines, through the pressure value measured by the first measurer 300, that the resection tool 3 is excessively close to the expander 200.

The resection-possible section D2 indicates a case in which the magnitude of the pressure measured by the first measurer 300 is between the first set pressure P1 and the second set pressure P2. That is, the resection-possible section D2 corresponds to a section in which the controller 410 determines, through the pressure value measured by the first measurer 300, that the resection tool 3 is positioned at an appropriate distance from the expander 200.

In a case in which the length of the patient's stomach is short and a portion of the expander 200 is not inserted into the stomach 2, the controller 410 excludes the first measuring member 310 of the first measurer 300 measuring a pressure value of the corresponding expander 200 from targets for determination. More specifically, the controller 410 distinguishes between the first measuring member 310 disposed inside the stomach 2 and measuring the same measurement value as the initial pressure P0 inside the stomach 2 and the first measuring member 310 disposed outside the stomach 2 and measuring a different measurement value from the initial pressure P0. The controller 410 determines that the first measurer 300 measuring the same measurement value as the initial pressure P0 inside the stomach 2 is in the approach-necessary section D1 and excludes other first measurers 300 from targets for determination.

The controller 410 may, through the measurement values of the plurality of first measuring members 310, determine whether the expander 200 inserted into the stomach 2 is correctly in close contact with the inner wall of the stomach 2. More specifically, in the case in which the expander 200 is inserted into the stomach, the controller 410 may compare magnitudes of pressure values separately measured by the plurality of first measuring members 310 disposed along the same circumference of the expander 200 to determine whether the expander 200 inserted into the stomach 2 is correctly in close contact with the inner wall of the stomach 2. That is, in a case in which a difference between measurement values of the first measuring members 310 in contact with the inner wall of the stomach 2 among the plurality of first measuring members 310 disposed along the same circumference of the expander 200 is within a set magnitude, the controller 410 determines that the expander 200 is correctly in close contact with the inner wall of the stomach 2, and in a case in which a difference between measurement values measured by any one pair of first measuring members 310 in contact with the inner wall of the stomach 2 is the set magnitude or more, the controller 410 determines that the expander 200 is not correctly in close contact with the inner wall of the stomach 2. Here, the set magnitude of the pressure value may be changed in various ways according to user settings.

The display 420 visually displays information determined by the controller 410 to the user. Accordingly, an example of the display 420 may be a display module or the like configured to output image information. The display 420 displays different colors according to the type of resection position section D determined by the controller 410. As the first measuring member 310 of the first measurer 300 is provided as a plurality of first measuring members 310, the display 420 may individually display information about each of the first measuring members 310.

The display 420 according to one embodiment of the present disclosure displays a first color C1 for the first measuring members 310 excluded from the targets for determination by the controller 410, displays a second color C2 for the first measuring members 310 determined as being in the approach-necessary section D1 by the controller 410, displays a third color C3 for the first measuring members 310 determined as being in the separation-necessary section D3 by the controller 410, and displays a fourth color C4 for the first measuring members 310 determined as being in the resection-possible section D2 by the controller 410. The first color C1 to the fourth color C4 may be changed in various ways within the range of the types of colors being distinguishable from each other.

In a case in which the controller 410 determines that the expander 200 is not in close contact with the inner wall of the stomach 2 as will be described below, the display 420 may generate a warning signal. In this case, examples of the warning signal may be an auditory signal such as an alarm sound or a visual signal of a color different from the first color C1 to the fourth color C4.

Hereinafter, the operation of the sleeve gastrectomy aid device 1 according to one embodiment of the present disclosure will be described in detail.

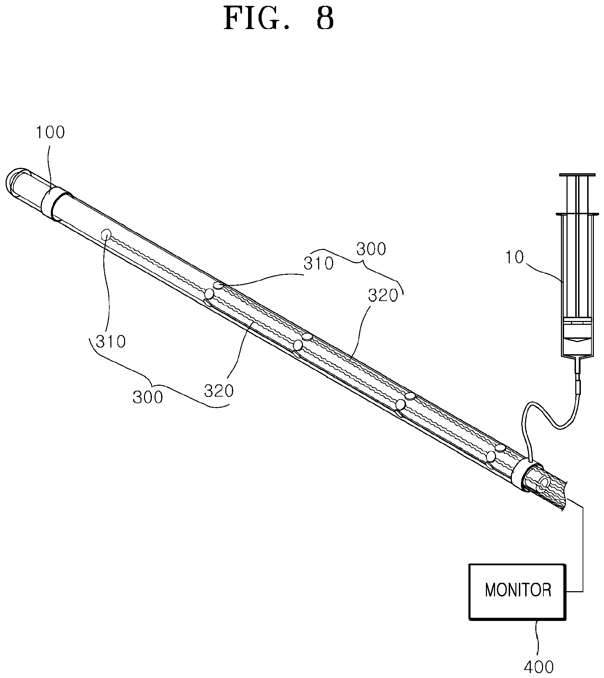

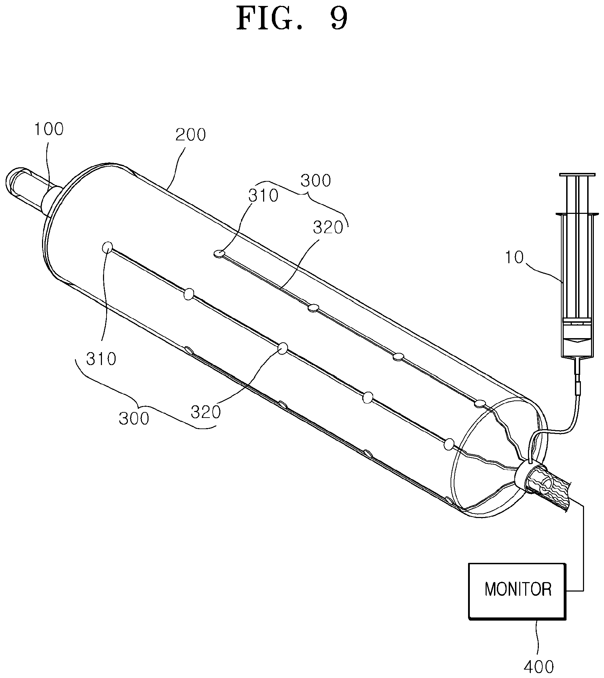

FIGS. 8 and 9 are operation diagrams schematically illustrating an operation process of the sleeve gastrectomy aid device according to one embodiment of the present disclosure.

Referring to FIGS. 8 and 9, the guide 100 inserted through the oral cavity or nasal cavity of a patient is inserted into the stomach 2 via the esophagus. The guide 100 inserted into the stomach 2 has an end disposed to face the pylorus of the stomach 2 and disposed close to an inner side surface of the stomach 2. In this case, the shape of the guide 100 may be changed corresponding to the curvature or the like of the stomach 2. !

Then, air is injected into the expander 200 through the injector 10 to expand the volume of the expander 200. In this case, both ends of the expander 200 are in close contact with and fixed to an outer side surface of the guide 100, and an outflow of air from inside the expander 200 is prevented. Accordingly, the expander 200 may maintain a state in which the volume thereof is expanded. Like a balloon, the expander 200 expands in a form in which a distance between two points arbitrarily set on a surface between both ends of the expander 200, which are in close contact with the outer peripheral surface of the guide 100, increases.

In such a process, the shape of the first measurer 300 is changed in accordance with a change in volume of the expander 200.

More specifically, as the volume of the expander 200 increases, a distance between adjacent first measuring members 310 increases.

As the distance between the adjacent first measuring members 310 increases, the pair of first measuring members 310 generate tension in the connector 320.

The connector 320 is extended as much as an amount of change in the distance between the adjacent first measuring members 310 in a direction parallel to the tension and offsets the tension applied thereto. Accordingly, the connector 320 may be prevented from breaking due to tension.

Then, when the volume of the expander 200 is reduced again and the distance between the first measuring members 310 decreases, the connector 320 is restored to its original form due to an elastic restoring force of the expander 200.

Through a pressure value measured by the first measurer 300, the monitor 400 provides resection position information to the user.

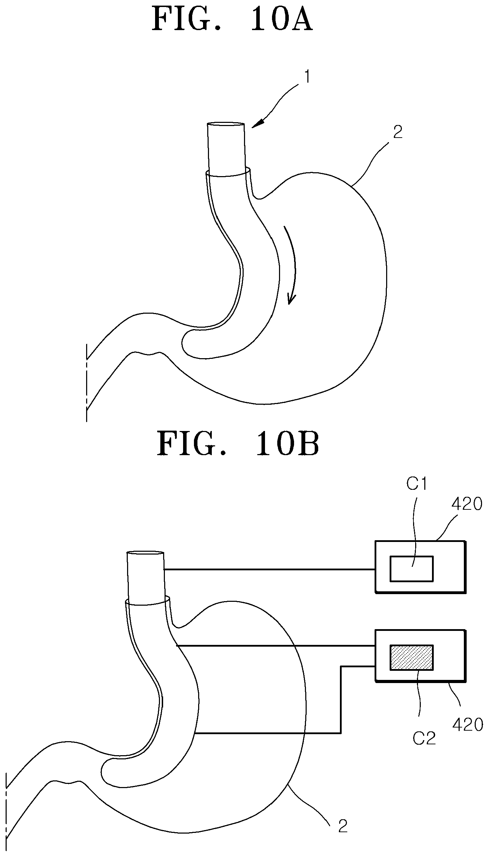

FIGS. 10A and 10B are operation diagrams schematically illustrating operation states of the controller in the process of inserting a guide according to one embodiment of the present disclosure.

Referring to FIGS. 10A and 10B, first, in the state in which the guide 100 is inserted into the stomach 2, the controller 410 calculates each of the number of first measuring members 310 disposed inside the stomach 2 and measuring the same measurement value as the initial pressure P0 inside the stomach 2 and the number of first measuring members 310 disposed outside the stomach 2 and measuring a different measurement value from the initial pressure P0. Then, the controller 410 determines that the first measuring members 310 measuring the same measurement value as the initial pressure P0 inside the stomach 2 are in the approach-necessary section D1 and excludes other first measuring members 310 from targets for determination.

The display 420 displays the information determined by the controller 410 to the outside. That is, as illustrated in FIG. 10, the display 420 displays the first color C1 for the first measuring members 310 excluded from the targets for determination by the controller 410 and displays the second color C2 for the first measuring members 310 determined as being in the approach-necessary section D1 by the controller 410.

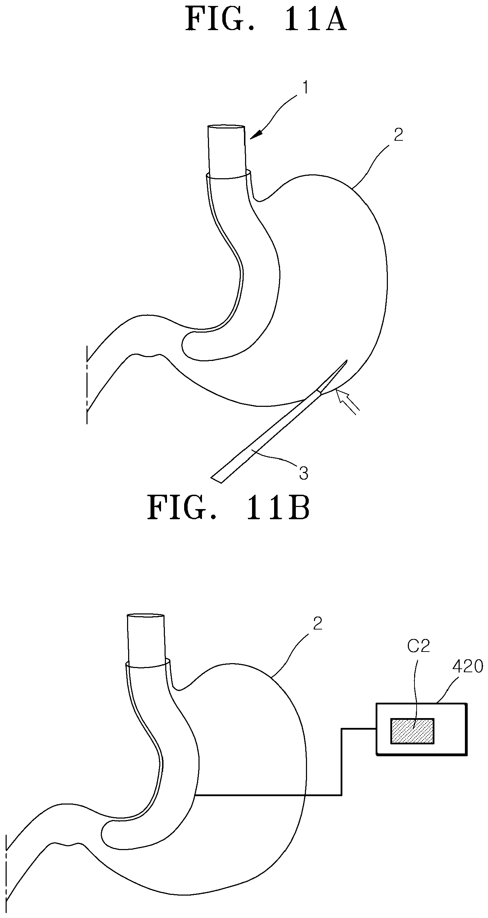

FIGS. 11A and 11B are operation diagrams schematically illustrating operation states of the controller in an approach-necessary section according to one embodiment of the present disclosure.

Referring to FIGS. 11A and 11B, the controller 410 determines resection position information of the resection tool 3 through a change in the pressure value measured by the first measurer 300, and the display 420 displays the determined information to the outside.

More specifically, as illustrated in FIG. 11A, in a case in which the resection tool 3 presses the stomach 2 in a state in which the resection tool 3 is further away from the expander 200 than the distances from the expander 200 to the resection tool 3 that allow the user to form the remaining stomach 2 with a target size, the pressure measured by the first measuring member 310 is lower than the first set pressure P1.

The controller 410 determines that the pressure value measured by the first measuring member 310 is in the approach-necessary section D 1 and transmits the corresponding information to the display 420. In this case, the controller 410 may use an average of pressure values measured by the remaining first measuring members 310 not excluded from the targets for determination among the plurality of first measuring members 310, that is, the first measuring members 310 determined to be in the approach-necessary section D1 in FIGS. 10A and 10B.

By displaying the second color C2 to the outside, the display 420 visually provides information indicating that the resection tool 3 is excessively far away from the expander 200 and needs to be closer to the expander 200.

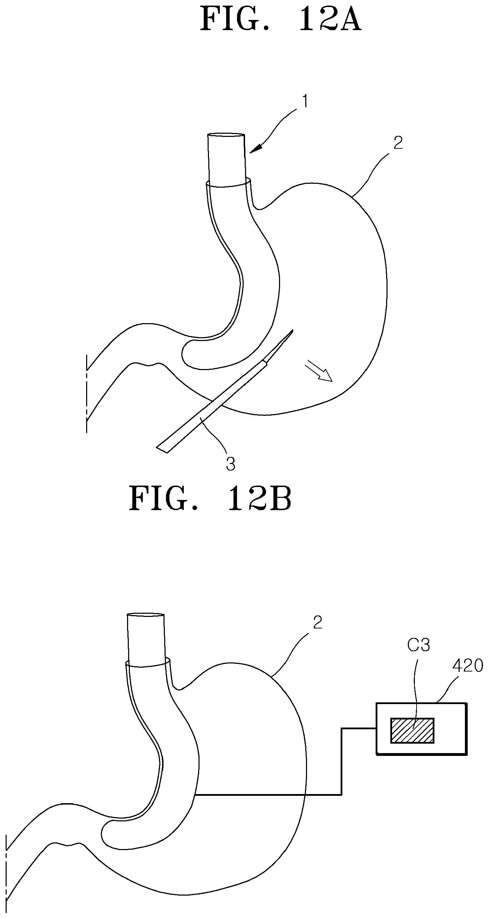

FIGS. 12A and 12B are operation diagrams schematically illustrating operation states of the controller in a separation-necessary section according to one embodiment of the present disclosure.

Referring to FIGS. 12A and 12B, in a case in which the resection tool 3 presses the stomach 2 in a state in which the resection tool 3 is closer to the expander 200 than the distances from the expander 200 to the resection tool 3 that allow the user to form the remaining stomach 2 with a target size, the pressure measured by the first measuring member 310 is higher than the second set pressure P2.

The controller 410 determines that the pressure value measured by the first measuring member 310 is in the separation-necessary section D3 and transmits the corresponding information to the display 420.

By displaying the third color C3 to the outside, the display 420 visually provides information indicating that the resection tool 3 is excessively far away from the expander 200 and needs to be further from the expander 200.

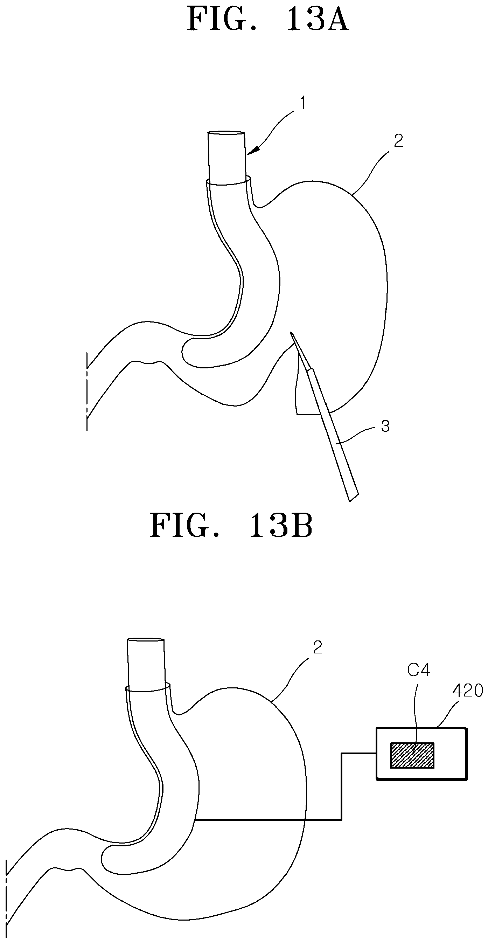

FIGS. 13A and 13B are operation diagrams schematically illustrating operation states of the controller in a resection-possible section according to one embodiment of the present disclosure.

Referring to FIGS. 13A and 13B, in a case in which the resection tool 3 presses the stomach 2 in a state in which the resection tool 3 is placed within the range of the distances from the expander 200 to the resection tool 3 that allow the user to form the remaining stomach 2 with a target size, the pressure value measured by the first measuring member 310 is between the first set pressure P1 and the second set pressure P2.

The controller 410 determines that the pressure value measured by the first measuring member 310 is in the resection-possible section D2 and transmits the corresponding information to the display 420.

By displaying the fourth color C4 to the outside, the display 420 visually provides information indicating that the resection tool 3 is placed at an appropriate distance from the expander 200 and thus resection may be performed using the resection tool 3.

Meanwhile, after the expander 200 is inserted into the stomach 2, the controller 410 may, through the measurement values of the plurality of first measuring members 310 disposed along the same circumference of the expander 200, determine whether the expander 200 inserted into the stomach 2 is correctly in close contact with the inner wall of the stomach 2.

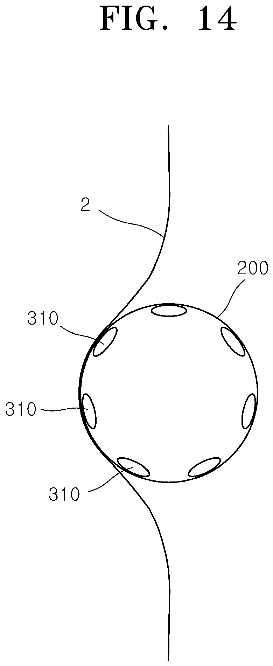

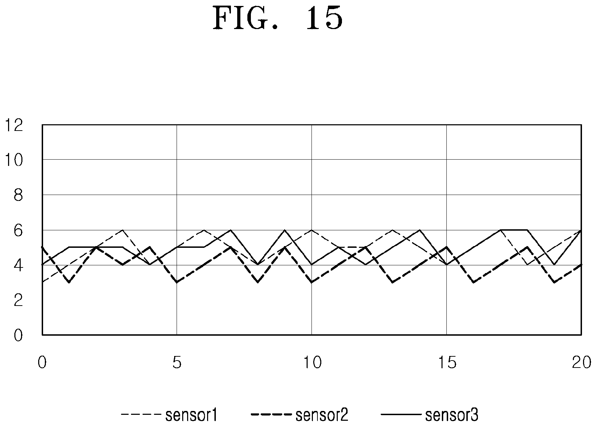

FIG. 14 is a view illustrating a state in which an expander is in close contact with an inner wall of the stomach according to one embodiment of the present disclosure, and FIG. 15 is a view illustrating pressure values measured by a plurality of first measuring members disposed along the same circumference of the expander in the state of FIG. 14.

Referring to FIGS. 14 and 15, in a case in which the expander 200 inserted into the stomach 2 is completely in close contact with the inner wall of the stomach 2, among the plurality of first measuring members 310 disposed along the same circumference of the expander 200, the first measuring members 310 in contact with the inner wall of the stomach 2 output measurement values within the set magnitude due to a uniform pressing force, and the controller 410 determines that the expander 200 is correctly in close contact with the inner wall of the stomach 2.

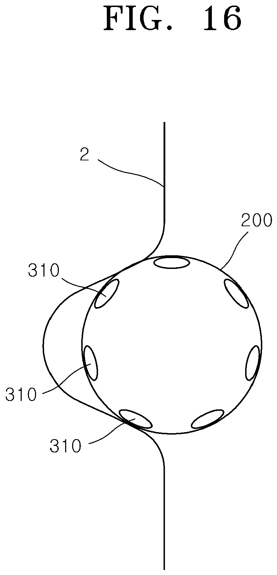

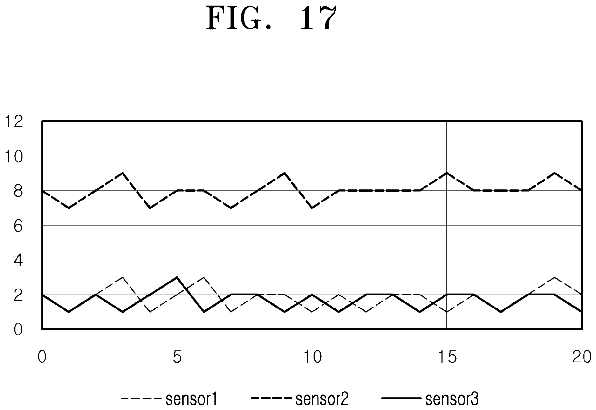

FIG. 16 is a view illustrating a state in which the expander is not in close contact with the inner wall of the stomach according to one embodiment of the present disclosure, and FIG. 17 is a view illustrating pressure values measured by the plurality of first measuring members disposed along the same circumference of the expander in the state of FIG. 16.

Referring to FIGS. 16 and 17, in a case in which the expander 200 inserted into the stomach 2 is not completely in close contact with the inner wall of the stomach 2, among the plurality of first measuring members 310 disposed along the same circumference of the expander 200, the first measuring members 310 in contact with the inner wall of the stomach 2 output different measurement values due to a non-uniform pressing force.

In a case in which a difference between measurement values measured by any one pair of first measuring members 310 in contact with the inner wall of the stomach 2 is the set magnitude or more, the controller 410 determines that the expander 200 is not correctly in close contact with the inner wall of the stomach 2. In the case in which it is determined by the controller 410 that the expander 200 is not correctly in close contact with the inner wall of the stomach 2, the display 420 generates a warning signal to allow the user to recognize that the expander 200 is not correctly in close contact with the inner wall of the stomach 2.

Hereinafter, a configuration of a sleeve gastrectomy aid device 1' according to another embodiment of the present disclosure will be described.

In this process, description overlapping with the above description of the sleeve gastrectomy aid device 1 according to one embodiment of the present disclosure will be omitted.

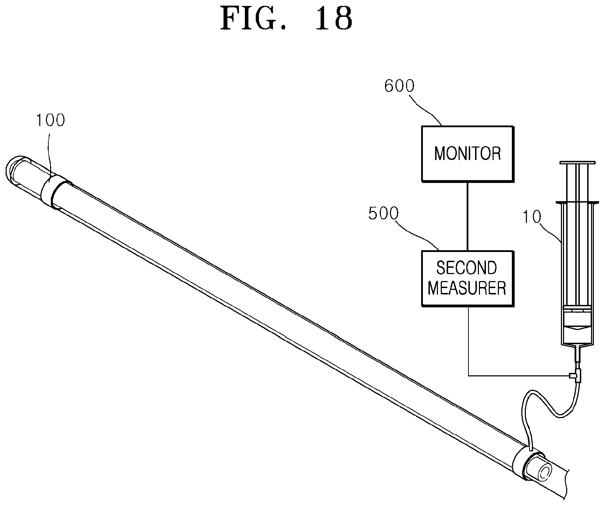

FIG. 18 is a perspective view schematically illustrating a configuration of a sleeve gastrectomy aid device according to another embodiment of the present disclosure.

Referring to FIG. 18, the sleeve gastrectomy aid device 1' according to another embodiment of the present disclosure includes a guide 100, an expander 200, a second measurer 500, and a monitor 600.

The second measurer 500 measures the air pressure inside the expander 200. More specifically, the second measurer 500 is provided to measure a value of the air pressure inside the expander 200 that changes due to pressure applied to the expander 200 as a resection tool 3 presses a stomach 2 during resection surgery. The second measurer 500 according to one embodiment of the present disclosure may be formed in the form of a pressure sensor connected to an injector 10 communicating with the expander 200 and configured to measure the pressure of air injected into the expander 200 through the injector 10. Accordingly, since the second measurer 500 can measure the pressure applied to the expander 200 from outside the expander 200, the second measurer 500 may be easily installed and managed. A pressure gauge that can provide a measured pressure value in the form of visual information to the user may be installed in the second measurer 500.

The monitor 600 is connected to the second measurer 500, determines resection position information through a pressure value measured by the second measurer 500, and provides the determined resection position information to the user. Here, an example of the resection position information may be information on a distance from the expander 200 to the resection tool 3.

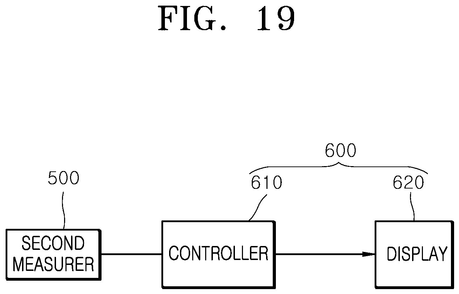

FIG. 19 is a flowchart schematically illustrating a configuration of a monitor according to another embodiment of the present disclosure.

Referring to FIG. 19, the monitor 600 according to another embodiment of the present disclosure includes a controller 610 and a display 620.

The controller 610 determines resection position information based on a pressure value measured by the second measurer 500. More specifically, the controller 610 converts the pressure value measured by the second measurer 500 into information on the distance from the expander 200 to the resection tool 3 to determine the resection position information of the resection tool 3. That is, by determining resection position sections D according to the magnitude of the pressure value measured by the second measurer 500, the controller 610 determines the resection position information. An example of the controller 610 according to another embodiment of the present disclosure may be a microprocessor, a computer, or the like connected to the second measurer 500 to receive the measured pressure value from the second measurer 500. The controller 610 may receive the measured pressure value from the second measurer 500 via a wire through a cable or the like or may receive the measured pressure value from the second measurer 500 through a separate wireless communication module.

The resection position sections D refer to sections obtained by dividing pressure values according to the sizes thereof, based on a value of an initial pressure P0 inside the stomach 2 that is measured by the second measurer 500 right after insertion of the guide 100. More specifically, the resection position sections D refer to sections between a pair of different pressure values among sections of pressures measured by the second measurer 500 as the position at which the stomach 2 is pressed or resected by the resection tool 3 is changed after the insertion of the guide 100.

The resection position sections D include an approach-necessary section D1, a resection-possible section D2, and a separation-necessary section D3.

The approach-necessary section D1 indicates a case in which the magnitude of the pressure measured by the second measurer 500 is the initial pressure P0 or higher and a first set pressure P1 or lower. Here, the first set pressure P1 is a pressure value measured at the maximum distance among distances from the expander 200 to the resection tool 3 that allow the user to form the remaining stomach 2 with a target size. The first set pressure P1 may be changed in various ways according to the shape, size, and the like of the stomach 2. That is, the approach-necessary section D1 corresponds to a section in which the controller 410 determines, through the pressure value measured by the second measurer 500, that the resection tool 3 is excessively far away from the expander 200.

The separation-necessary section D3 indicates a case in which the magnitude of the pressure measured by the second measurer 500 is a second set pressure P2 or higher. Here, the second set pressure P2 is a pressure value measured at the minimum distance among the distances from the expander 200 to the resection tool 3 that allow the user to form the remaining stomach 2 with a target size. The second set pressure P2 may be changed in various ways according to the shape, size, and the like of the stomach 2. More specifically, the second set pressure P2 indicates a pressure lower than or equal to the maximum pressure that can be measured by the second measurer 500 or a pressure Pmax at which the mucosa of the stomach 2 ruptures. That is, the separation-necessary section D3 corresponds to a section in which the controller 610 determines, through the pressure value measured by the second measurer 500, that the resection tool 3 is excessively close to the expander 200.

The resection-possible section D2 indicates a case in which the magnitude of the pressure measured by the second measurer 500 is between the first set pressure P1 and the second set pressure P2. That is, the resection-possible section D2 corresponds to a section in which the controller 610 determines, through the pressure value measured by the second measurer 500, that the resection tool 3 is positioned at an appropriate distance from the expander 200.

The display 620 visually displays information determined by the controller 610 to the user. Accordingly, an example of the display 620 may be a display module or the like configured to output image information. The display 620 displays different colors according to the type of resection position section D determined by the controller 610.

The display 620 according to another embodiment of the present disclosure displays a second color C2 in a case in which the controller 610 determines that the pressure value measured by the second measurer 500 is in the approach-necessary section D1, displays a third color C3 in a case in which the controller 610 determines that the pressure value measured by the second measurer 500 is in the separation-necessary section D3, and displays a fourth color C4 in a case in which the controller 610 determines that the pressure value measured by the second measurer 500 is in the resection-possible section D2. The second color C2 to the fourth color C4 may be changed in various ways within the range of the types of colors being distinguishable from each other.

Hereinafter, an operation process of the sleeve gastrectomy aid device 4 according to another embodiment of the present disclosure will be described in detail.

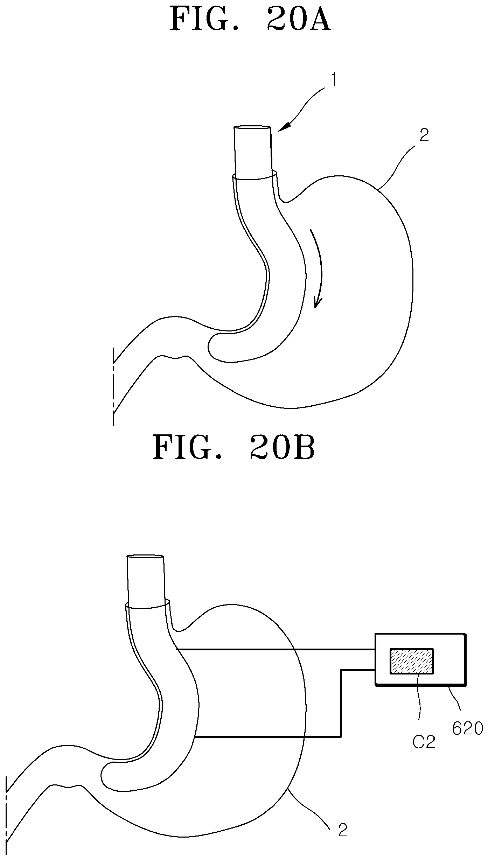

FIGS. 20A and 20B are operation diagrams schematically illustrating operation states of a controller in the process of inserting a guide according to another embodiment of the present disclosure.

Referring to FIGS. 20A and 20B, as the second measurer 500 measures the initial pressure P0 inside the stomach 2 right after the guide 100 is inserted into the stomach 2, the controller 610 determines that the pressure value measured by the second measurer 500 is in the approach-necessary section D1, and the display 620 displays the second color C2.

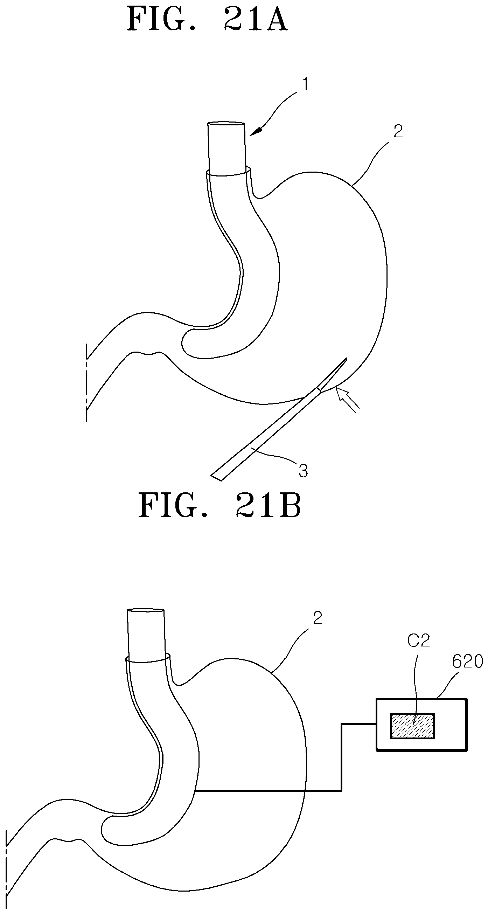

FIGS. 21A and 21B are operation diagrams schematically illustrating operation states of the controller in an approach-necessary section according to another embodiment of the present disclosure.

Referring to FIGS. 21A and 21B, in a case in which the resection tool 3 presses the stomach 2 in a state in which the resection tool 3 is further away from the expander 200 than the distances from the expander 200 to the resection tool 3 that allow the user to form the remaining stomach 2 with a target size, the pressure measured by the second measurer 500 is lower than the first set pressure P1.

The controller 610 determines that the pressure value measured by the second measurer 500 is in the approach-necessary section D1 and transmits the corresponding information to the display 620.

By displaying the second color C2 to the outside, the display 620 visually provides information indicating that the resection tool 3 is excessively far away from the expander 200 and needs to be closer to the expander 200.

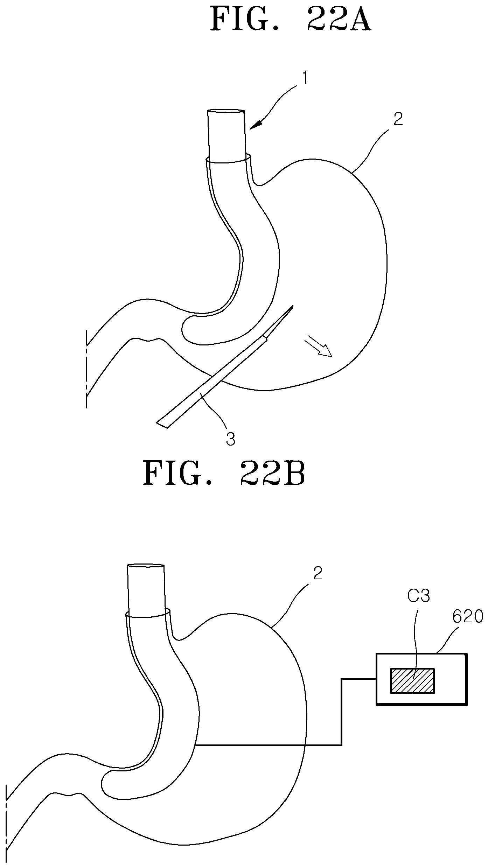

FIGS. 22A and 22B are operation diagrams schematically illustrating operation states of the controller in a separation-necessary section according to another embodiment of the present disclosure.

Referring to FIGS. 22A and 22B, in a case in which the resection tool 3 presses the stomach 2 in a state in which the resection tool 3 is closer to the expander 200 than the distances from the expander 200 to the resection tool 3 that allow the user to form the remaining stomach 2 with a target size, the pressure measured by the second measurer 500 is higher than the second set pressure P2.

The controller 610 determines that the pressure value measured by the second measurer 500 is in the separation-necessary section D3 and transmits the corresponding information to the display 620.

By displaying the third color C3 to the outside, the display 620 visually provides information indicating that the resection tool 3 is excessively far away from the expander 200 and needs to be further from the expander 200.

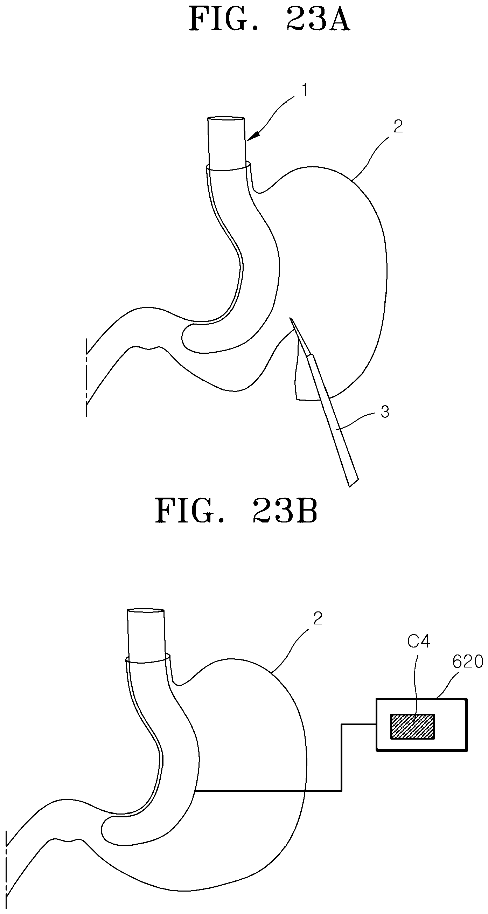

FIGS. 23A and 23B are operation diagrams schematically illustrating operation states of the controller in a resection-possible section according to another embodiment of the present disclosure.

Referring to FIGS. 23A and 23B, in a case in which the resection tool 3 presses the stomach 2 in a state in which the resection tool 3 is placed within the range of the distances from the expander 200 to the resection tool 3 that allow the user to form the remaining stomach 2 with a target size, the pressure value measured by the second measurer 500 is between the first set pressure P1 and the second set pressure P2.

The controller 610 determines that the pressure value measured by the second measurer 500 is in the resection-possible section D2 and transmits the corresponding information to the display 620.

By displaying the fourth color C4 to the outside, the display 620 visually provides information indicating that the resection tool 3 is placed at an appropriate distance from the expander 200 and thus resection may be performed using the resection tool 3.

The present disclosure has been described with reference to the embodiments illustrated in the drawings, but the embodiments are merely illustrative, and those of ordinary skill in the art should understand that various modifications and other equivalent embodiments are possible from the embodiments described herein.

Therefore, the technical scope of the present disclosure should be defined by the claims below.

## Claims

1. A sleeve gastrectomy aid device comprising:
a guide configured to be inserted into a stomach of a patient;
an expander configured to be disposed outside the guide and have a volume that changes according to an amount of air therein;
a first measurer configured to measure a pressure applied to the expander and have a shape that changes in accordance with a change in volume of the expander; and
a monitor configured to be connected to the first measurer and provide resection position information through a value of the pressure measured by the first measurer.

2. The sleeve gastrectomy aid device of claim 1, wherein the first measurer includes:
a plurality of first measuring members configured to be attached to an inner side surface of the expander; and
a connector configured to be connected to the first measuring members adjacent to each other and expand and contract according to a distance between the first measuring members adjacent to each other.

3. The sleeve gastrectomy aid device of claim 2, wherein the first measurer extends in a longitudinal direction of the expander and is provided as a plurality of first measurers, and the plurality of first measurers are spaced apart in a circumferential direction of the expander.

4. The sleeve gastrectomy aid device of claim 3, wherein the plurality of first measuring members provided in each of the first measurers are disposed to be spaced apart at predetermined intervals in the longitudinal direction of the expander.

5. The sleeve gastrectomy aid device of claim 4, wherein the first measuring members provided in the first measurers, which are different from each other, are disposed along the same circumference of the expander.

6. The sleeve gastrectomy aid device of claim 2, wherein the connector extends in a zigzag shape in a longitudinal direction thereof.

7. The sleeve gastrectomy aid device of claim 2, wherein the connector includes a plurality of connecting members connected to each other in a lattice form and each having a diameter expanding in a direction parallel to a direction in which an external force is applied.

8. The sleeve gastrectomy aid device of claim 2, wherein the connector is made of a material capable of conducting current.

9. The sleeve gastrectomy aid device of claim 1, wherein the resection position information is information on a distance from the expander to a resection tool.

10. The sleeve gastrectomy aid device of claim 1, wherein the monitor includes:
a controller configured to determine the resection position information based on the value of the pressure measured by the first measurer; and
a display configured to display the information determined by the controller to the outside.

11. The sleeve gastrectomy aid device of claim 10, wherein the controller determines resection position sections according to a magnitude of the value of the pressure measured by the first measurer.

12. The sleeve gastrectomy aid device of claim 11, wherein the resection position sections include:
an approach-necessary section indicating a case in which the magnitude of the pressure measured by the first measurer is lower than or equal to a first set pressure;
a separation-necessary section indicating a case in which the magnitude of the pressure measured by the first measurer is higher than or equal to a second set pressure; and
a resection-possible section indicating that the magnitude of the pressure measured by the first measurer is between the first set pressure and the second set pressure.

13. The sleeve gastrectomy aid device of claim 11, wherein the display displays different colors according to the resection position sections.

14. A sleeve gastrectomy aid device comprising:
a guide configured to be inserted into a stomach of a patient;
an expander configured to be disposed outside the guide and have a volume that changes according to an amount of air therein;
a second measurer configured to measure an air pressure inside the expander; and
a monitor configured to be connected to the second measurer and provide resection position information through a value of the pressure measured by the second measurer.

15. The sleeve gastrectomy aid device of claim 14, wherein the second measurer is connected to an injector configured to inject air into the expander.

16. The sleeve gastrectomy aid device of claim 14, wherein the resection position information is information on a distance from the expander to a resection tool.

17. The sleeve gastrectomy aid device of claim 14, wherein the monitor includes:
a controller configured to determine the resection position information based on the value of the pressure measured by the second measurer; and
a display configured to display the information determined by the controller to the outside.

18. The sleeve gastrectomy aid device of claim 17, wherein the controller determines resection position sections according to a magnitude of the value of the pressure measured by the second measurer.

19. The sleeve gastrectomy aid device of claim 18, wherein the resection position sections include:
an approach-necessary section indicating a case in which the magnitude of the pressure measured by the second measurer is lower than or equal to a first set pressure;
a separation-necessary section indicating a case in which the magnitude of the pressure measured by the second measurer is higher than or equal to a second set pressure; and
a resection-possible section indicating that the magnitude of the pressure measured by the second measurer is between the first set pressure and the second set pressure.

20. The sleeve gastrectomy aid device of claim 18, wherein the display displays different colors according to the resection position sections.
